# FASCICULE DE BREVET EUROPEEN

(11) **EP 2 334 255 B1**
(45) Date de publication et mention de la délivrance du brevet: **28.03.2012**
(21) Numéro de dépôt: 09741348.8
(22) Date de dépôt: 01.09.2009
(51) Int. Cl.: A61F 2/00

(54) **Implant prothétique de soutènement sous-urétral à goussets**
Prothesenimplantat zur suburethralen Unterstützung mit Verstärkungen
Prosthetic implant for suburethral support with gussets

(30) Priorité: 01.09.2008 FR 0804788
(43) Date de publication de la demande: 22.06.2011
(73) Titulaire: Compagnie de Recherche en Composants, Implants et Materiels pour L'Application Clinique, 38200 Vienne (FR)
(72) Inventeur: MARTIN, Laure-Cécile, F-69360 Ternay (FR); RICOL, Jean-Paul, Gilbert, F-69210 Saint Germain Sur L'arbresle (FR)
(74) Mandataire: Croonenbroek, Thomas Jakob
(86) Numéro de dépôt international: PCT/FR2009/051650
(87) Numéro de publication internationale: WO 2010/023417

(56) Documents cités:
- EP-A- 1 911 415
- WO-A-2006/048885
- US-A- 5 922 026
- US-A1- 2008 021 356

## Description

### DOMAINE TECHNIQUE

La présente invention se rapporte au domaine technique général des dispositifs implantables chirurgicalement pour soutenir l'urètre, en vue de traiter l'incontinence urinaire, et notamment en vue de traiter l'incontinence urinaire d'effort féminine.

La présente invention concerne plus particulièrement un implant prothétique de soutènement sous-urétral comprenant un élément de soutènement sous-urétral de forme allongée s'étendant longitudinalement entre une première et une deuxième extrémité, ladite première extrémité au moins présentant une première et une deuxième face opposées, telle que définie dans la revendication 1.

L'invention concerne également un procédé de fabrication d'un implant prothétique de soutènement sous-urétral comprenant une étape de fabrication ou de fourniture d'un élément de soutènement sous-urétral de forme allongée s'étendant longitudinalement entre une première et une deuxième extrémité, ladite première extrémité au moins présentant une première et une deuxième face opposées, telle que définie dans la revendication 11.

### TECHNIQUE ANTERIEURE

L'incontinence urinaire correspond à une perte involontaire d'urine à travers l'urètre. Cette perte involontaire provient généralement du fait que les tissus responsables de la retenue des urines ne sont plus en mesure d'exercer des forces suffisantes pour s'opposer aux forces d'expulsion des urines. L'incontinence urinaire d'effort correspond en particulier à une perte d'urine non précédée de sensation de besoin, et qui ne se produit pas au repos mais pendant l'effort.

Parmi les traitements chirurgicaux proposés pour corriger l'incontinence urinaire, on connaît en particulier le traitement par soutènement urétral à l'aide d'une bandelette. La bandelette en question est mise en place chirurgicalement sans tension, sous la partie moyenne de l'urètre, et est destinée à supporter ce dernier pendant l'effort pour empêcher ainsi toute fuite d'urine. On distingue plusieurs types de bandelettes associées à des voies chirurgicales différentes. On connaît ainsi la bandelette TVT (Tension-free Vaginal Tape), associée au traitement chirurgical par la voie rétro-pubienne et la bandelette TOT (Trans-Obturator Tape) associée au traitement chirurgical par voie trans-obturatrice, pour ne citer que certaines parmi les plus répandues.

La méthode utilisant la bandelette TVT s'avère toutefois relativement délicate à mettre en oeuvre et présente des risques opératoires non négligeables, dans la mesure où il existe notamment un risque de perforation de la vessie lors de la manipulation des aiguilles utilisées pour la pose de la bandelette. C'est la raison pour laquelle il est indispensable, avec cette méthode chirurgicale, de pratiquer une cystoscopie. Des conséquences plus graves ont même parfois été constatées, telles qu'une perforation de l'artère iliaque ou encore de l'intestin grêle, entraînant des complications viscérales parfois mortelles.

Le traitement chirurgical utilisant la bandelette TOT présente *a priori* moins de risques de perforation d'organes nobles, mais n'élimine cependant pas complètement le risque de complications vasculaires en raison de la longueur du trajet occupé par la bandelette au sein du corps.

Afin de remédier aux inconvénients des méthodes TVT et TOT exposées dans ce qui précède, il a été proposé récemment par la demanderesse une méthode mini-invasive mettant en oeuvre une bandelette sous-urétrale comprenant à chacune de ses extrémités un gousset, comme décrit dans le document EP 1911415.

Cette bandelette sous-urétrale est relativement courte et est destinée à être posée par voie vaginale sans tension, le maintien en position de la bandelette au sein du corps de la patiente étant obtenu par simple appui et friction des goussets sur les tissus biologiques.

Cette bandelette donne globalement satisfaction, dans la mesure notamment où elle est de construction particulièrement simple et bon marché, tout en étant efficace et peu invasive.

Elle n'en présente pas moins un certain nombre d'inconvénients. Ainsi, il a été observé dans quelques cas et dans certaines conditions un maintien non optimal de la bandelette au sein des tissus biologiques, susceptible d'engendrer des récidives et/ou des érosions vaginales, la bandelette non correctement tenue ayant tendance à frotter contre le vagin ce qui provoque des érosions.

Le document US 5922026 décrit une bandelette comprenant deux goussets, sur des faces opposées de la bandelette mais situés aux extrémités opposées de la bandelette.

### EXPOSE DE L'INVENTION

Les objets assignés à l'invention visent en conséquence à proposer un nouvel implant prothétique de soutènement sous-urétral qui ne présente pas les inconvénients mentionnés précédemment et qui permette de corriger de manière efficace les incontinences urinaires d'effort en diminuant les risques de complications opératoires et de récidive, tout en étant de construction particulièrement simple, fiable et bon marché.

Un autre objet de l'invention vise à proposer un nouvel implant prothétique de soutènement sous-urétral dont la conception permet un excellent maintien en position dudit implant au sein du corps du patient.

Un autre objet de l'invention vise à proposer un nouvel implant prothétique de soutènement sous-urétral de construction particulièrement simple.

Un autre objet de l'invention vise à proposer un nouvel implant prothétique de soutènement sous-urétral qui soit particulièrement facile à mettre en place, tout en étant extrêmement stable en position au sein du corps du patient une fois posé.

Un autre objet de l'invention vise à proposer un nouvel implant prothétique de soutènement sous-urétral particulièrement simple et bon marché à fabriquer.

Un autre objet de l'invention vise à proposer un nouvel implant prothétique de soutènement sous-urétral capable d'être mis en place par voie vaginale exclusivement.

Un autre objet de l'invention vise à proposer un nouveau procédé de fabrication d'un implant prothétique de soutènement sous-urétral qui permet d'obtenir un implant prothétique de soutènement sous-urétral particulièrement efficace et de construction simple et bon marché.

Un autre objet de l'invention vise à proposer un nouveau procédé de fabrication d'un implant prothétique de soutènement sous-urétral dont la mise en oeuvre, en particulier dans un contexte industriel, est particulièrement facile, rapide et bon marché.

Les objets assignés à l'invention sont atteints à l'aide d'un implant prothétique de soutènement sous-urétral comprenant un élément de soutènement sous-urétral de forme allongée s'étendant longitudinalement entre une première et une deuxième extrémité, ladite première extrémité au moins présentant une première et une deuxième face opposées, ledit implant comprenant au moins un premier gousset qui fait saillie du côté de ladite première face de la première extrémité et étant caractérisé en ce que ledit élément de soutènement sous-urétral comprend un deuxième gousset qui fait saillie du côté de ladite deuxième face de la première extrémité, lesdits premier et deuxième goussets étant conçus pour accrocher ladite première extrémité au sein des tissus biologiques du patient.

Les objets assignés à l'invention sont également atteints à l'aide d'un procédé de fabrication d'un implant prothétique de soutènement sous-urétral comprenant une étape de fabrication ou de fourniture d'un élément de soutènement sous-urétral de forme allongée s'étendant longitudinalement entre une première et une deuxième extrémité, ladite première extrémité au moins présentant une première et une deuxième face opposées, ledit procédé comprenant une étape d'association audit élément de soutènement sous-urétral d'un premier gousset qui fait saillie du côté de ladite première face de la première extrémité et étant caractérisé en ce qu'il comprend un deuxième gousset qui fait saillie du côté de ladite deuxième face de la première extrémité, lesdits premier et deuxième goussets étant conçus pour accrocher ladite première extrémité au sein des tissus biologiques du patient.

### DESCRIPTIF SOMMAIRE DES DESSINS

D'autres avantages et objets de l'invention apparaîtront plus en détail à la lecture de la description qui suit, ainsi qu'à l'aide des dessins annexés fournis à titre purement illustratifs et non limitatifs, dans lesquels :
- La figure 1 illustre, selon une vue de dessus, un implant prothétique conforme à un premier mode de réalisation de l'invention, dans un état précédant sa mise en forme terminale.
- La figure 2 illustre, selon une vue schématique, un implant prothétique conforme à l'invention, qui correspond à l'implant de la figure 1 dans sa mise en forme terminale.
- La figure 3 illustre, selon une vue schématique en coupe longitudinale, l'implant prothétique de la figure 2.
- La figure 4 illustre, selon une vue de dessus, un implant conforme à un deuxième mode de réalisation de l'invention, dans un état précédant sa mise en forme terminale.
- Les figures 5 à 11 illustrent différentes étapes de mise en place chirurgicale de l'implant prothétique des figures 1 à 3.
- La figure 12 illustre l'implant prothétique des figures 1 à 3 accompagné d'un instrument chirurgical destiné à en faciliter sa mise en place.

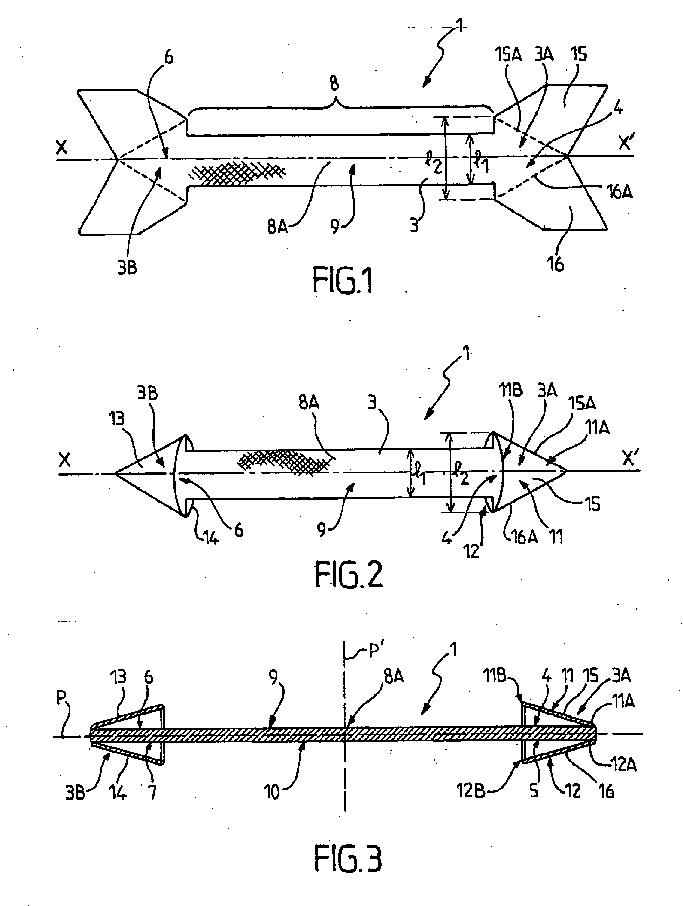

- Les figures 2 à 4 et 12 montrent un implant prothétique 1 de soutènement sous-urétral conforme à l'invention destiné à être implanté dans le corps d'un patient en vue de réaliser un soutènement de l'urètre permettant de corriger l'incontinence urinaire, et notamment l'incontinence urinaire d'effort chez la femme.

### MEILLEURE MANIERE DE REALISER L'INVENTION

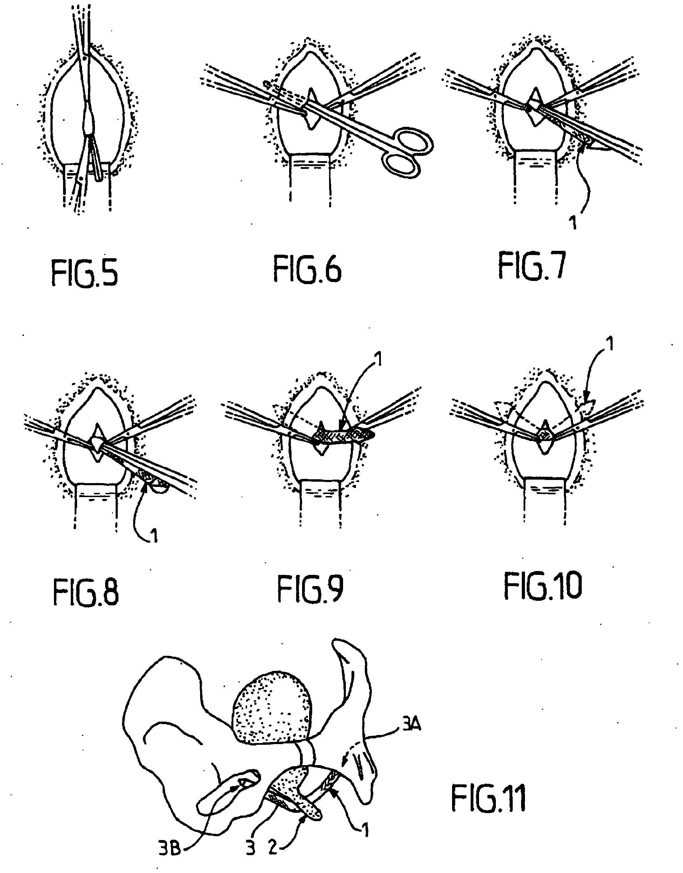

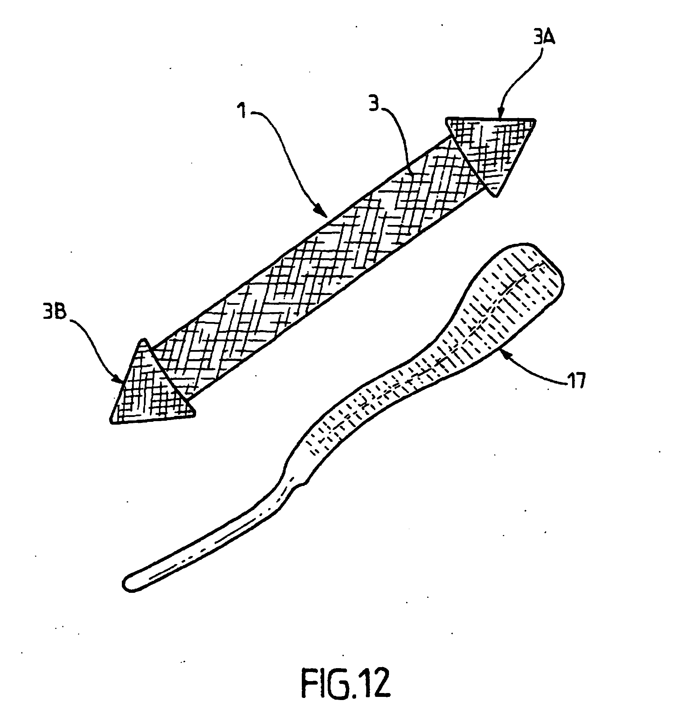

Le terme « *implant prothétique* » désigne ici une prothèse préfabriquée, destinée à être introduite à l'intérieur du corps du patient, de préférence par voie chirurgicale. La figure 11 illustre l'implant prothétique 1 dans sa position fonctionnelle, c'est-à-dire la position dans laquelle il va permettre de soutenir l'urètre 2 et d'empêcher ainsi les fuites involontaires d'urine. Selon l'invention, l'implant prothétique de soutènement sous-urétral 1 comprend un élément de soutènement sous-urétral 3 de forme allongée s'étendant longitudinalement entre une première et une deuxième extrémité 3A, 3B. De préférence, l'élément de soutènement sous-urétral 3 est formé par une bandelette textile, telle que celle illustrée sur la figure 12. Cette bandelette est de préférence réalisée en polypropylène tricoté et présente avantageusement une structure ouverte, c'est-à-dire pourvue d'ouvertures délimitées par différents jeux de fils. Selon l'invention, l'élément de soutènement sous-urétral 3 est sensiblement souple, et plus précisément est suffisamment souple et flexible pour d'une part éviter un phénomène de cisaillement des tissus biologiques après sa mise en place au sein du corps, et d'autre part lui permettre de suivre les éventuels mouvements des tissus biologiques environnants après son implantation.

Conformément à l'invention, la première extrémité 3A au moins présente une première et une deuxième face 4, 5 opposées. Avantageusement, la deuxième extrémité 3B présente elle aussi une première et une deuxième face 6, 7 opposées. La bandelette textile formant avantageusement l'élément de soutènement sous-urétral 3 se compose ainsi de préférence d'une bande principale 8 sensiblement rectangulaire et présentant elle-même une première et une deuxième face opposées 9, 10. Les premières faces 4, 6 respectives des première et deuxième extrémités 3A, 3B s'étendent avantageusement dans le prolongement de la première face 9 de la bande principale 8, dans le même plan que cette dernière, tandis que les deuxièmes faces 5, 7 respectives des première et deuxième extrémités 3A, 3B s'étendent dans le prolongement de la deuxième face 10 de la bande principale 8, dans le même plan que ladite deuxième face 10.

Conformément à l'invention, l'élément de soutènement sous urétral 3 comprend au moins un premier gousset 11 qui fait saillie du côté de la première face 4 de la première extrémité 3A et un deuxième gousset 12 qui fait saillie du côté de la deuxième face 5 de la première extrémité 3A, lesdits premier et deuxième goussets 11, 12 étant conçus pour accrocher la première extrémité 3A de l'élément de soutènement sous-urétral 3 au sein des tissus biologiques du patient. En d'autres termes, la première extrémité 3A s'étend dans un plan moyen P et présente des faces 4, 5 qui sont situées de part et d'autre dudit plan P, le premier gousset 11 étant situé du côté de la première face 4, tandis que le deuxième gousset 12 est situé de l'autre côté du plan P, du côté de la deuxième face 5. Dans la variante illustrée aux figures 1 à 3, le premier gousset 11 s'élève sensiblement au droit de la face 4, de façon à former une protubérance à la surface de la première extrémité 3A du côté de sa première face 4. De même, le deuxième gousset 12 s'élève au droit de la deuxième face 5, de façon à former une protubérance à la surface de la première extrémité 3A, mais cette fois du côté de sa deuxième face 5. Il n'est toutefois pas nécessaire que les goussets s'élèvent strictement au droit des faces 4, 5 de la première extrémité 3A, le principal étant qu'ils se situent de chaque côté du plan moyen d'extension P dans lequel s'inscrit sensiblement l'extrémité 3A en question. De préférence, la bandelette dans son ensemble s'étend selon ledit plan P.

Par « *gousset* » on désigne ici une petite poche, qui, en l'occurrence, s'ouvre préférentiellement du côté de la partie centrale 8A de la bande principale 8.

Les premier et deuxième goussets 11, 12 forment ainsi des pochettes souples qui se déploient à la surface de ladite première extrémité 3A pour constituer un moyen d'encrage, par appui et friction, de ladite première extrémité 3A au sein des tissus biologiques du patient, de façon à maintenir l'élément de soutènement 3 dans une position déterminée. La demanderesse a ainsi mis en évidence que le recours à deux goussets 11, 12 disposés de part et d'autre de la première extrémité 3A permettait d'obtenir un excellent accrochage, fiable et confortable pour le patient, de ladite première extrémité 3A de l'élément de soutènement 3. Les goussets 11, 12 permettent ainsi à eux seuls le maintien en position de l'élément de soutènement 3, par auto-accrochage aux tissus biologiques environnants, sans qu'il ne soit besoin de recourir à des moyens externes additionnels du genre agrafes. En outre, le recours à des goussets 11, 12 en tant que d'accrochage est de nature à faciliter la fabrication de l'élément de soutènement 3, comme cela sera expliqué plus en détails dans ce qui suit.

Le recours à des goussets permet également de faciliter l'implantation, dans la mesure où les goussets 11, 12 ont la faculté de pouvoir s'aplatir contre les faces 4, 5 dont ils sont respectivement issus au moment de l'introduction, ce qui facilite cette dernière. À l'inverse, les goussets 11, 12 ont également la capacité de se déployer verticalement à partir des faces 4, 5 dont ils sont respectivement issus, comme illustré aux figures 2 et 3, afin de remplir une fonction d'accrochage optimisé au sein des tissus, évitant le désengagement intempestif de l'implant une fois ce dernier posé dans le corps du patient.

Avantageusement, les premier et deuxième goussets 11, 12 sont sensiblement identiques et sont disposés de façon symétrique relativement à la première extrémité 3A, c'est-à-dire qu'ils sont disposés de façon symétrique par rapport au plan moyen P dans lequel s'étend la bandelette formant l'élément de soutènement sous-urétral 3. Dans ce cas, les premier et deuxième goussets 11, 12 sont avantageusement situés au droit l'un de l'autre.

De préférence, chacun desdits premier et deuxième goussets 11, 12 présente une face avant pointue 11A, 12A respective pour faciliter l'introduction de la première extrémité 3A au sein des tissus biologiques du patient.

De préférence, chacun desdits premier et deuxième goussets 11, 12 présente également une face arrière respective 11B, 12B élargie pour former une butée anti-retour permettant l'accrochage de la première extrémité 3A au sein desdits tissus biologiques.

En d'autres termes, les premier et deuxième goussets 11, 12 sont avantageusement profilés en forme de pointe vers l'extérieur de l'élément de soutènement 3, de manière à faciliter la pénétration et le déplacement dudit élément de soutènement 3 dans l'espace de dissection. À l'arrière de la pointe, chaque gousset 11, 12 présente une base élargie destinée à venir en appui contre les tissus biologiques et empêcher ainsi le glissement de la bandelette textile formant l'élément de soutènement 3. La première extrémité 3A équipée de ses premier et deuxième goussets 11, 12 fonctionne donc à la manière d'un parapluie, puisqu'elle présente une capacité accrue de pénétration dans les tissus dans un sens (sens de l'implantation) tout en présentant une certaine résistance à revenir en arrière, dans le sens de l'explantation, ce qui garantit un excellent maintien de l'élément de soutènement sous-urétral 3 dans le corps du patient.

Avantageusement, ledit élément de soutènement sous-urétral 3 comprend également un troisième gousset 13 qui fait saillie de la première face 6 de la deuxième extrémité 3B et un quatrième gousset 14 qui fait saillie de la deuxième face 7 de la deuxième extrémité 3B, lesdits troisième et quatrième goussets 13, 14 étant conçus, à l'instar des premier et deuxième goussets 11, 12, pour sensiblement accrocher ladite deuxième extrémité 3B de l'élément de soutènement sous-urétral 3 au sein des tissus biologiques du patient.

De préférence, les troisième et quatrième goussets 13, 14 sont construits de façon symétrique aux premier et deuxième goussets 11, 12.

Cela signifie que l'élément de soutènement sous-urétral 3 présente avantageusement non seulement une symétrie par rapport au plan d'extension P mais également une symétrie par rapport au plan médian P' qui est perpendiculaire au plan P et qui passe par le centre 8A de l'élément de soutènement sous-urétral 3. La construction et les fonctions de la deuxième extrémité 3B et des goussets associés 13, 14 sont ainsi identiques à celles de la première extrémité 3A et des goussets 11, 12 associés, de sorte qu'il n'apparaît pas nécessaire de décrire plus en détails la deuxième extrémité 3B et ses goussets 13, 14 compte-tenu du caractère symétrique de l'élément de soutènement sous-urétral 3.

Avantageusement, la première extrémité 3A, ainsi que de préférence la deuxième extrémité 3B, sont, au moins en partie, plus larges que le reste de l'élément de soutènement sous-urétral 3. Par exemple, comme illustré aux figures 1 à 3, l'élément de soutènement sous-urétral 3 peut se présenter sous la forme d'une bande centrale 8 rectangulaire de largeur l₁ (mesurée dans le plan moyen d'extension P) sensiblement constante, la première extrémité 3A présentant au moins une portion de largeur l₂ supérieure à la largeur l₁ de la bande centrale 8, comme illustré aux figures. Par exemple, la première extrémité 3A se présente avantageusement sous la forme d'une pièce plate en forme de triangle dont la base, de longueur l₂, s'étend perpendiculairement à l'axe longitudinal X-X' d'extension de l'élément de soutènement 3, un sommet du triangle étant disposé sur ledit axe longitudinal X-X'. Dans ce cas, la première extrémité 3A présente une forme de pointe qui facilite l'introduction au sein des tissus de l'élément de soutènement 3 tout en procurant, dans le plan moyen d'extension P, un effet anti-retour obtenu grâce au décrochement latéral que forme l'extrémité 3A par rapport à la bande centrale 8.

Ainsi, dans cette variante préférentielle de réalisation illustrée aux figures 1 à 3, la première extrémité 3A est pourvue de moyens anti-retour à la fois dans le plan moyen d'extension P (lesdits moyens anti-retour étant dans ce cas formés par l'élargissement de la première extrémité 3A par rapport au reste de l'élément de soutènement 3) et dans le plan P' perpendiculaire au plan P (lesdits moyens anti-retour étant formés dans ce plan par les premier et deuxième goussets 11, 12).

De préférence, au moins l'un desdits premier et deuxième goussets 11, 12, et de préférence les deux premier et deuxième goussets 11, 12, s'étendent sur toute la face 4, 5 de la première extrémité 3A dont ils sont issus. Cela signifie en particulier, dans le cas des variantes illustrées aux figures, que les goussets 11, 12 s'étendent également au droit des zones élargies (de largeur l₂) de la première extrémité 3A, de sorte que lesdits goussets 11, 12 renforcent ainsi l'effet anti-retour procuré par ledit élargissement dans le plan P, en y ajoutant une composante supplémentaire d'accrochage dans le plan P'.

Avantageusement, lesdits premier et deuxième goussets 11, 12 viennent de matière avec ladite première extrémité 3A, tout comme lesdits troisième et quatrième goussets 13, 14 viennent avantageusement de matière avec ladite deuxième extrémité 3B. L'un des principaux intérêts de recourir à des goussets en tant que moyen d'accrochage réside justement dans la possibilité de réaliser aisément des goussets sur chaque face de l'élément de soutènement à partir d'une pièce d'un seul tenant.

Dans les variantes illustrées aux figures, lesdits premier et deuxième goussets 11, 12 sont ainsi avantageusement réalisés à partir de la bandelette textile formant l'élément de soutènement sous-urétral 3. Il est cependant tout à fait envisageable que chaque gousset 11, 12, 13, 14 soit formé par une pièce distincte et indépendante rapportée et fixée sur la bandelette textile par tout procédé approprié (couture, soudure, collage ou autre).

Avantageusement, comme illustré à la figure 1, ladite bandelette textile comprend une première et une deuxième aile 15, 16 disposées à la première extrémité 3A, et plus précisément de part et d'autre de ladite extrémité 3A, de façon symétrique relativement à l'axe d'extension longitudinal X-X' de l'élément de soutènement 3. La première aile 15 est avantageusement rabattue sur la première face 4 de la première extrémité 3A pour former le premier gousset 11, tandis-que la deuxième aile 16 est rabattue sur la deuxième face 15 de la deuxième extrémité 3A pour former le deuxième gousset 12. Par exemple, la première aile 15 est pliée selon une ligne de pliure 15A (représentée en pointillés sur la figure 1) de façon à être rabattue sur la première face 4, ladite première aile 15 étant ensuite avantageusement cousue (ou soudée, ou collée) à la première face 4 pour former le premier gousset 11. De même, la deuxième aile 16 est pliée selon une ligne de pliure 16A (représentée en pointillés sur la figure 1) de façon à être rabattue sur la deuxième face 5, ladite deuxième aile 16 étant avantageusement cousue sur la deuxième face 5 pour former le deuxième gousset 12.

Il est ainsi possible, à partir d'une pièce textile unique d'un seul tenant (illustrée à la figure 1), de réaliser de façon particulièrement simple, à l'aide de simples opérations de pliage et de couture, un élément de soutènement 3 conforme à l'invention, qui présente un excellent maintien au sein du corps du patient.

Selon la variante illustrée aux figures 1 à 3, l'élément de soutènement sous-urétral 3 se présente ainsi avantageusement sous la forme d'une bande centrale 8 rectangulaire prolongée par deux triangles formant respectivement les première et deuxième extrémités 3A, 3B, la base de chacun desdits triangles s'étendant perpendiculairement à la bande centrale 8, tandis que les deux autres côtés du triangle forment les lignes de pliure 15A, 16A à partir desquelles s'étendent latéralement (par rapport à l'axe X-X') les ailes 15, 16. ces dernières sont leurs lignes respectives de pliure 15A, 16A de façon à venir épouser sur toute la longueur l'autre ligne de pliure 16A, 15A et y être cousues, de préférence sur toute leur longueur.

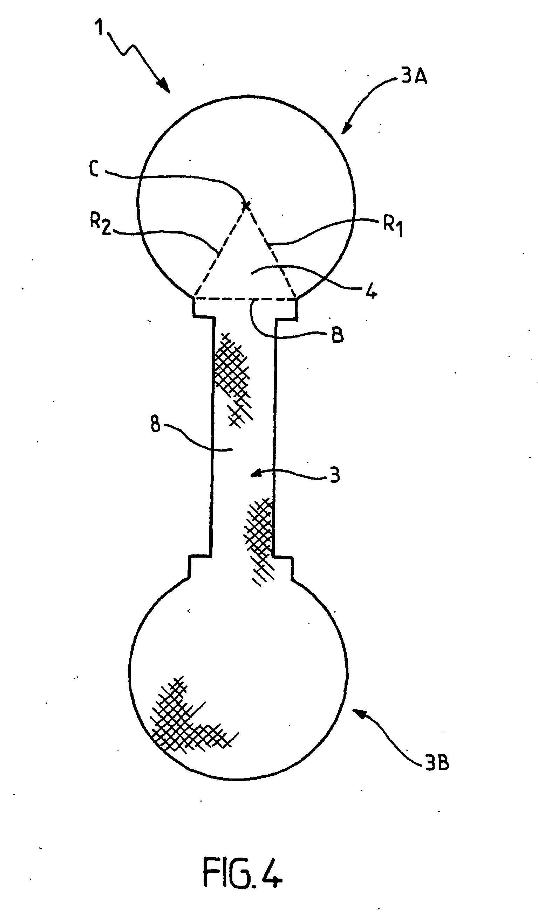

Dans la variante de la figure 4, la bandelette textile se présente sous la forme d'une pièce d'un seul tenant composée d'une bande centrale rectangulaire 8 prolongée à chacune de ses extrémités 3A, 3B par une plaque sensiblement circulaire, de centre C pour ce qui concerne la première extrémité 3A. Une découpe de ladite plaque circulaire de centre C est réalisée selon la ligne correspondant au rayon R₁. On obtient ainsi une plaque circulaire de centre C pourvu d'une encoche sur toute la longueur de son rayon R₁. La plaque circulaire ainsi fendue peut alors être mise en forme de cône de sommet C, ledit cône englobant le triangle délimité par les lignes pointillées R1, R₂ et B et étant rattaché à la bandelette par le côté R₂. Les goussets 11, 12 sont donc dans ce cas formés par un cône unique qui entoure les faces 4, 5 de l'extrémité 3A et forme du côté de la face 4 le premier gousset 11 et du côté de la face 5 le deuxième gousset 12.

Avantageusement, la longueur globale de l'élément de soutènement sous-urétral 3 est sensiblement comprise entre 90 et 140 mm, et de préférence de l'ordre de 110 mm.

Avantageusement, la largeur moyenne de l'élément de soutènement sous-urétral est sensiblement comprise entre 9 et 20 mm, et de préférence est de l'ordre de 12 mm.

La mise en place de l'implant prothétique 1 conforme à l'invention est avantageusement effectuée de la manière suivante.

Tout d'abord, la patiente est installée en position gynécologique, jambes à 90 degrés de flexion. La description des gestes opératoires effectués dans ce qui suit se rapporte à cette position. Une incision verticale médiane profonde d'environ 2,5 cm est réalisée sur la paroi vaginale intérieure à environ 1,5cm du méat urinaire, comme illustré sur la figure 5. A partir de cette incision, on introduit latéralement des ciseaux entre paroi vaginale et urètrè, perpendiculairement à la branche ischio-pubienne, en allant chercher le contact avec cette branche osseuse. Cette étape est illustrée à la figure 6. Ce trajet est alors poursuivi en arrière de cette branche osseuse en gardant le même axe perpendiculaire et jusqu'à ouvrir l'aponévrose pelvienne. Sur place, les ciseaux sont alors ouverts délicatement puis referm"s avant d'être retirés. Les mêmes gestes sont réalisés de chaque côté. Comme illustré à la figure 7, la pointe des ciseaux courbes est ensuite enfilée dans l'un des goussets de l'extrémité de la bandelette de polypropylène formant l'implant prothétique de soutènement sous-urétral 1 conforme à l'invention.

Ainsi gainée, ladite bandelette est glissée dans le trajet réalisé dans les étapes précédentes, emmenant l'extrémité 3A de la bandelette jusqu'au niveau des muscles obturateurs, au travers de l'aponévrose et sans traverser la membrane obturatrice. Le passage de la pointe des ciseaux avec un léger ressaut à travers l'aponévrose doit être ressenti. Il garantit le bon positionnement de chaque extrémité 3A, 3B de la bandelette. En variante, comme illustré à la figure 8, il est possible d'utiliser un ancillaire 17 pour retrouver le trajet de dissection, l'ancillaire 17 servant de guide au ciseau pour emmener la bandelette jusqu'au niveau des muscles obturateurs, au-travers de l'aponévrose et sans traverser la membrane obturatrice. A l'issue de cette étape, l'ancillaire 17 est alors retiré.

Comme illustré à la figure 9, les ciseaux sont enfin retirés, laissant la bandelette dans le trajet. Le même geste est réalisé de manière identique de l'autre côté. La bandelette est ainsi simplement posée sans tension, comme illustré aux figures 10 et 11. Si la tension n'est pas suffisante, la réintroduction des ciseaux au fond du trajet, dans l'un des goussets de l'extrémité concernée, est facile et permettra le repositionnement adéquat.

Les goussets 11, 12, 13, 14 assurent un effet anti retour optimal qui permet le maintien en position stable de la bandelette au sein du corps du patient.

Cette méthode chirurgicale est mini-invasive, et réduit donc le temps opératoire et les risques de complications associés. Ces risques de complications sont d'autant plus réduits que l'implant une fois posé se trouve à distance du paquet vasculo-nerveux et que la membrane obturatrice n'est pas perforée. Les douleurs névralgiques postopératoires sont ainsi sensiblement diminuées.

En outre, la quantité de matériau implanté est minimisée, compte-tenu des petites dimensions de la bandelette en regard des bandelettes mises en oeuvre dans les techniques connues antérieurement.

La bandelette conforme à l'invention est ainsi particulièrement adaptée à la technique opératoire décrite dans ce qui précède, qui est une technique d'introduction par voie vaginale exclusive.

Enfin, l'invention concerne également un procédé de fabrication d'un implant prothétique 1 de soutènement sous-urétral, et en particulier d'un implant prothétique 1 conforme à l'invention.

Le procédé de fabrication selon l'invention comprend :
- une étape de fabrication ou de fourniture d'un élément de soutènement sous-urétral 3 de forme allongée s'étendant longitudinalement entre une première et une deuxième extrémité 3A, 3B, ladite première extrémité 3A au moins présentant une première et une deuxième face opposées 4, 5,
- et une étape d'association audit élément de soutènement sous-urétral 3 d'un premier gousset 11 qui fait saillie du côté de ladite première face 4 de la première extrémité 3A et d'un deuxième gousset 12 qui fait saillie du côté de ladite deuxième face 5 de la première extrémité 3A, lesdits premier et deuxième gousset 11, 12 étant conçus pour accrocher ladite première extrémité 3A au sein des tissus biologiques du patient.

Avantageusement, ladite étape de fabrication ou de fourniture de l'élément de soutènement sous-urétral 3 comprend la fabrication et la fourniture d'une bandelette textile destinée à former ledit élément de soutènement sous-urétral 3, ladite bandelette comprenant elle-même une première et une deuxième aile 15, 16 disposées à la première extrémité 3A, ladite étape d'association comprenant une opération de formation des goussets 11, 12 dans laquelle la première aile 15 est rabattue sur la première face 4 de la première extrémité 3A pour former le premier gousset 11, tandis que la deuxième aile 16 est rabattue sur la deuxième face 5 pour former le deuxième gousset 12.

Le procédé conforme à l'invention permet ainsi de fabriquer de manière extrêmement simple, rapide et facilement industrialisable une prothèse présentant une excellente tenue au sein du corps du patient, à partir d'une seule pièce textile découpée de manière à adopter une forme conforme à celle de la figure 1 par exemple.

### POSSIBILITE D'APPLICATION INDUSTRIELLE

L'invention trouve son application industrielle dans la conception, la fabrication et l'utilisation d'implants chirurgicaux.

## Revendications

1. - Implant prothétique (1) de soutènement sous-urétral comprenant un élément de soutènement sous-urétral (3) de forme allongée s'étendant longitudinalement entre une première et une deuxième extrémité (3A, 3B), ladite première extrémité (3A) au moins présentant une première et une deuxième face opposées (4, 5), ledit implant (1)
comprenant au moins un premier gousset (11) qui fait saillie du côté de ladite première face (4) de la première extrémité (3A) et étant **caractérisé en ce que** ledit élément de soutènement sous-urétral (3) comprend un deuxième gousset (12).
qui fait saillie du côté de ladite deuxième face (5) de la première extrémité (3A), lesdits premier et deuxième goussets (11, 12) étant conçus pour accrocher ladite première extrémité (3A) au sein des tissus biologiques du patient.

2. - Implant (1) selon la revendication 1 **caractérisé en ce que** ladite deuxième extrémité (3B) présente une première et une deuxième face opposées (6, 7), ledit élément de soutènement sous-urétral (3) comprenant au moins un troisième gousset (13) qui fait saillie du côté de ladite première face (6) de la deuxième extrémité (3B) et un quatrième gousset (14) qui fait saillie du côté de ladite deuxième face (7) de la deuxième extrémité (3B), lesdits troisième et quatrième goussets (13, 14) étant conçus pour sensiblement accrocher ladite deuxième extrémité (3B) au sein des tissus biologiques du patient.

3. - Implant (1) selon la revendication 1 ou 2 **caractérisé en ce que** lesdits premier et deuxième goussets (11, 12) sont sensiblement identiques et sont disposés de façon symétrique relativement à la première extrémité (3A).

4. - Implant (1) selon l'une des revendications 1 à 3 **caractérisé en ce que** chacun desdits premier et deuxième goussets (11, 12) présente une face avant pointue (11A) pour faciliter l'introduction de la première extrémité (3A) au sein des tissus biologiques du patient et une face arrière (11 B) élargie pour former une butée anti-retour permettant l'accrochage de ladite première extrémité (3A) au sein desdits tissus biologiques.

5. - implant (1) selon l'une des revendication 1 à 4 **caractérisé en ce que** lesdites première et deuxième extrémité (3A, 3B) sont, au moins en partie, plus large que le reste de l'élément de soutènement sous-urétral (3).

6. - Implant (1) selon l'une des revendications 1 à 5 **caractérisé en ce que** lesdits premier et deuxième goussets (11, 12) viennent de matière avec ladite première extrémité (3A).

7. - Implant (1) selon l'une des revendications 1 à 6 **caractérisé en ce que** l'élément de soutènement sous-urétral (3) est formé par une bandelette textile et **en ce que** lesdits premier et deuxième goussets (11, 12) sont réalisés à partir de ladite bandelette textile.

8. - Implant (1) selon la revendication 7 **caractérisé en ce que** la bandelette textile comprend une première et une deuxième aile (15, 16) disposées à la première extrémité (3A), la première aile (15) étant rabattue sur la première face (4) de la première extrémité (3A) pour former le premier gousset (11) tandis que la deuxième aile (16) est rabattue sur la deuxième face (5) de la première extrémité (3A) pour former le deuxième gousset (12).

9. - Implant (1) selon l'une des revendications précédentes **caractérisé en ce que** la longueur de l'élément de soutènement sous-urétral (3) est sensiblement comprise entre 90 et 140 mm, de préférence de l'ordre de 110 mm.

10. - Implant (1) selon l'une des revendications précédentes **caractérisé en ce que** la largeur de l'élément de soutènement sous-urétral (3) est sensiblement comprise entre 9 et 20 mm, de préférence de l'ordre de 12 mm.

11. - Procédé de fabrication d'un implant prothétique (1) de soutènement sous-urétral comprenant une étape de fabrication ou de fourniture d'un élément de soutènement sous-urétral (3) de forme allongée s'étendant longitudinalement entre une première et une deuxième extrémité (3A, 3B), ladite première extrémité (3A) au moins présentant une première et une deuxième face opposées (4, 5), ledit procédé comprenant une étape d'association audit élément de soutènement sous urétral (3) d'un premier gousset (11) qui fait saillie du côté de ladite première face (4) de la première extrémité (3A) et étant **caractérisé en ce qu'**il comprend un deuxième gousset (12) qui fait saillie du côté de ladite deuxième face (5) de la première extrémité (3A), lesdits premier et deuxième goussets (11, 12) étant conçus pour accrocher ladite première extrémité (3A) au sein des tissus biologiques du patient.

12. -Procédé de fabrication selon la revendication 11 **caractérisé en ce que** ladite étape de fabrication ou de fourniture de l'élément de soutènement sous-urétral (3) comprend la fabrication ou la fourniture d'une bandelette textile destinée à former ledit élément de soutènement sous-urétral (3), ladite bandelette comprenant une première et une deuxième aile (15, 16) disposées à la première extrémité (3A), ladite étape d'association comprenant une opération de formation des goussets (11, 12) dans laquelle la première aile (15) est rabattue sur la première face (4) de la première extrémité (3A) pour former le premier gousset (11) tandis que la deuxième aile (16) est rabattue sur la deuxième face (5) pour former le deuxième gousset (12).

## Claims

1. Prosthetic implant (1) for suburethral support comprising a suburethral supporting element (3) of elongate shape extending longitudinally between a first and a second end (3A, 3B), said first end (3A) at least having a first and a second opposite face (4, 5), said implant (1) comprising at least a first gusset (11) that protrudes from the side of said first face (4) of the first end (3A) and being **characterized in that** said suburethral supporting element (3) comprises a second gusset (12) that protrudes from the side of said second face (5) of the first end (3A), said first and second gussets (11, 12) being designed to hook said first end (3A) into the biological tissues of the patient.

2. Implant (1) according to Claim 1, **characterized in that** said second end (3B) has a first and a second opposite face (6, 7), said suburethral supporting element (3) comprising at least a third gusset (13) that protrudes from the side of said first face (6) of the second end (3B) and a fourth gusset (14) that protrudes from the side of said second face (7) of the second end (3B), said third and fourth gussets (13, 14) being designed to substantially hook said second end (3B) into the biological tissues of the patient.

3. Implant (1) according to Claim 1 or 2, **characterized in that** said first and second gussets (11, 12) are substantially identical and are placed symmetrically relatively to the first end (3A).

4. Implant (1) according to one of Claims 1 to 3, **characterized in that** each of said first and second gussets (11, 12) has a pointed front face (11A) in order to make it easier to insert the first end (3A) into the biological tissues of the patient and a rear face (11B) that is widened in order to form an anti-return abutment making it possible to hook said first end (3A) into said biological tissues.

5. Implant (1) according to one of Claims 1 to 4, **characterized in that** said first and second ends (3A, 3B) are, at least partly, wider than the rest of the suburethral supporting element (3).

6. Implant (1) according to one of Claims 1 to 5, **characterized in that** said first and second gussets (11, 12) are made in one piece with and of the same material as said first end (3A).

7. Implant (1) according to one of Claims 1 to 6, **characterized in that** the suburethral supporting element (3) is formed by a textile tape and **in that** said first and second gussets (11, 12) are made from said textile tape.

8. Implant (1) according to Claim 7, **characterized in that** the textile tape comprises a first and a second flange (15, 16) placed at the first end (3A), the first flange (15) being folded over the first face (4) of the first end (3A) in order to form the first gusset (11), while the second flange (16) is folded over the second face (5) of the first end (3A) in order to form the second gusset (12).

9. Implant (1) according to one of the preceding claims, **characterized in that** the length of the suburethral supporting element (3) is between 90 and 140 mm, preferably of the order of 110 mm.

10. Implant (1) according to one of the preceding claims, **characterized in that** the width of the suburethral supporting element (3) is substantially between 9 and 20 mm, preferably of the order of 12 mm.

11. Method for manufacturing a suburethral supporting prosthetic implant (1) comprising a step of manufacturing or of supplying a suburethral supporting element (3) of elongate shape extending longitudinally between a first and a second end (3A, 3B), said first end (3A) at least having a first and a second opposite face (4, 5), said method comprising a step of adjoining to said suburethral supporting element (3) a first gusset (11) that protrudes from the side of said first face (4) of the first end (3A) and being **characterized in that** it comprises a second gusset (12) that protrudes from the side of said second face (5) of the first end (3A), said first and second gussets (11, 12) being designed to hook said first end (3A) into the biological tissues of the patient.

12. Manufacturing method according to Claim 11, **characterized in that** said step of manufacturing or of supplying the suburethral supporting element (3) comprises the manufacture or the supply of a textile tape designed to form said suburethral supporting element (3), said tape comprising a first and a second flange (15, 16) placed at the first end (3A), said adjoining step comprising an operation of forming the gussets (11, 12) in which the first flange (15) is folded over the first face (4) of the first end (3A) in order to form the first gusset (11), while the second flange (16) is folded over the second face (5) in order to form the second gusset (12).

## Patentansprüche

1. Prothesenimplantat (1) zur suburethralen Unterstützung, umfassend ein länglich geformtes Element (3) zur suburethralen Unterstützung, das sich in Längsrichtung zwischen einem ersten und einem zweiten Ende (3A, 3B) erstreckt, wobei das erste Ende (3A) mindestens eine erste und eine zweite gegenüberliegende Stirnseite (4, 5) aufweist, wobei das Implantat (1) mindestens ein erstes Eckstück (11) umfasst, das auf der Seite der ersten Stirnseite (4) des ersten Endes (3A) hervorragt, und **dadurch gekennzeichnet ist, dass** das besagte Element (3) zur suburethralen Unterstützung ein zweites Eckstück (12) umfasst, das auf der Seite der zweiten Stirnseite (5) des ersten Endes (3A) hervorragt, wobei das erste und zweite Eckstück (11, 12) ausgelegt sind, das erste Ende (3A) im biologischen Gewebe des Patienten zu verhaken.

2. Implantat (1) nach Anspruch 1, **dadurch gekennzeichnet, dass** das zweite Ende (3B) eine erste und eine zweite gegenüberliegende Stirnseite (6, 7) aufweist, wobei das Element (3) zur suburethralen Unterstützung mindestens ein drittes Eckstück (13), das auf der Seite der ersten Stirnseite (6) des zweiten Endes (3B) hervorragt, und ein viertes Eckstück (14), das auf der Seite der zweiten Stirnseite (7) des zweiten Endes (3B) hervorragt, umfasst, wobei das dritte und vierte Eckstück (13, 14) ausgelegt sind, das zweite Ende (3B) im biologischen Gewebe des Patienten im Wesentlichen zu verhaken.

3. Implantat (1) nach Anspruch 1 oder 2, **dadurch gekennzeichnet, dass** das erste und zweite Eckstück (11, 12) im Wesentlichen identisch sind und im Verhältnis zum ersten Ende (3A) symmetrisch angeordnet sind.

4. Implantat (1) nach einem der Ansprüche 1 bis 3, **dadurch gekennzeichnet, dass** das erste und zweite Eckstück (11, 12) jeweils eine spitze Vorderseite (11A), um die Einführung des ersten Endes (3A) in das biologische Gewebe des Patienten zu erleichtern, und eine verbreiterte Rückseite (11B), um eine Rücklaufsperre zu bilden, die das Verhaken des ersten Endes (3A) in dem biologischen Gewebe ermöglicht, aufweisen.

5. Implantat (1) nach einem der Ansprüche 1 bis 4, **dadurch gekennzeichnet, dass** das erste und zweite Ende (3A, 3B) mindestens teilweise größer sind als der Rest des Elements (3) zur suburethralen Unterstützung.

6. Implantat (1) nach einem der Ansprüche 1 bis 5, **dadurch gekennzeichnet, dass** das erste und zweite Eckstück (11, 12) mit dem ersten Ende (3A) aus demselben Material einstückig geformt sind.

7. Implantat (1) nach einem der Ansprüche 1 bis 6, **dadurch gekennzeichnet, dass** das Element (3) zur suburethralen Unterstützung durch ein Stoffbändchen gebildet ist, und dass das erste und zweite Eckstück (11, 12) aus dem Stoffbändchen ausgebildet sind.

8. Implantat (1) nach Anspruch 7, **dadurch gekennzeichnet, dass** das Stoffbändchen einen ersten und einen zweiten Flügel (15, 16) umfasst, die an dem ersten Ende (3A) angeordnet sind, wobei der erste Flügel (15) auf die erste Stirnseite (4) des ersten Endes (3A) umgeschlagen ist, um das erste Eckstück (11) zu bilden, während der zweite Flügel (16) auf die zweite Stirnseite (5) des ersten Endes (3A) umgeschlagen ist, um das zweite Eckstück (12) zu bilden.

9. Implantat (1) nach einem der vorhergehenden Ansprüche, **dadurch gekennzeichnet, dass** die Länge des Elements (3) zur suburethralen Unterstützung im Wesentlichen zwischen 90 und 140 mm liegt, bevorzugt ungefähr 110 mm beträgt.

10. Implantat (1) nach einem der vorhergehenden Ansprüche, **dadurch gekennzeichnet, dass** die Breite des Elements (3) zur suburethralen Unterstützung im Wesentlichen zwischen 9 und 20 mm liegt, bevorzugt ungefähr 12 mm beträgt.

11. Verfahren zum Herstellen eines Prothesenimplantats (1) zur suburethralen Unterstützung, umfassend einen Schritt des Herstellens oder des Bereitstellens eines länglich geformten Elements (3) zur suburethralen Unterstützung, das sich in Längsrichtung zwischen einem ersten und einem zweiten Ende (3A, 3B) erstreckt, wobei das erste Ende (3A) mindestens eine erste und eine zweite gegenüberliegende Stirnseite (4, 5) aufweist, wobei das Verfahren einen Schritt des Verknüpfens mit dem Element (3) zur suburethralen Unterstützung eines ersten Eckstücks (11), das auf der Seite der ersten Stirnseite (4) des ersten Endes (3A) hervorragt, umfasst und **dadurch gekennzeichnet ist, dass** es ein zweites Eckstück (12) umfasst, das auf der Seite der zweiten Stirnseite (5) des ersten Endes (3A) hervorragt, wobei das erste und zweite Eckstück (11, 12) ausgelegt sind, das erste Ende (3A) im biologischen Gewebe des Patienten zu verhaken.

12. Herstellungsverfahren nach Anspruch 11, **dadurch gekennzeichnet, dass** der Schritt des Herstellens oder des Bereitstellens des Elements (3) zur suburethralen Unterstützung das Herstellen oder Bereitstellen eines Stoffbändchens umfasst, das dazu bestimmt ist, das Element (3) zur suburethralen Unterstützung zu bilden, wobei das Bändchen einen ersten und einen zweiten Flügel (15, 16) umfasst, die am ersten Ende (3A) angeordnet sind, wobei der Schritt des Verknüpfens einen Vorgang zum Bilden der Eckstücke (11, 12) umfasst, wobei der erste Flügel (15) auf die erste Stirnseite (4) des ersten Endes (3A) umgeschlagen wird, um das erste Eckstück (11) zu bilden, während der zweite Flügel (16) auf die zweite Stirnseite (5) umgeschlagen wird, um das zweite Eckstück (12) zu bilden.
